(19) Europäisches Patentamt
European Patent Office
Office européen des brevets

(11) Publication number: 0 024 348
B1

(12) # EUROPEAN PATENT SPECIFICATION

(45) Date of publication of patent specification: 13.11.85

(51) Int. Cl.⁴: C 07 D 309/30, A 61 K 31/365, C 07 C 59/48, C 07 C 69/73

(21) Application number: 80104807.5

(22) Date of filing: 14.08.80

(54) **Substituted 6-Phenethyl- and phenylethenyl-3,4,5,6-tetrahydro-4-hydroxytetrahydropyran-2-ones in the 4-R trans stereoisomeric forms and the corresponding dihydroxy acids, process for preparing and pharmaceutical composition comprising them.**

(30) Priority: 17.08.79 US 67574

(43) Date of publication of application:
04.03.81 Bulletin 81/09

(45) Publication of the grant of the patent:
13.11.85 Bulletin 85/46

(84) Designated Contracting States:
AT BE CH DE FR GB IT LI LU NL SE

(56) References cited:
EP-A-0 010 951
FR-A-2 392 016

LIEBIGS ANNALEN DER CHEMIE (1979) pages 484-491 Weinheim DE H.H. MEYER: "Synthesen von (−)-(S)- und (+)-(R)-Goniothalamins absolute Konfiguration des natürlichen (+)-Goniothalamins"

**The file contains technical information submitted after the application was filed and not included in this specification**

(73) Proprietor: MERCK & CO. INC.
126, East Lincoln Avenue P.O. Box 2000
Rahway New Jersey 07065 (US)

(72) Inventor: Willard, Alvin K.
1712 Jamison Court
Lansdale Pennsylvania 19446 (US)
Inventor: Cragoe, Edward J., Jr.
2211 Oak Terrace Drive
Lansdale Pennsylvania 19446 (US)
Inventor: Novello, Frederick C.
786 Bair Road
Berwyn Pennsylvania 19312 (US)
Inventor: Hoffman, William F.
740 Weikel Road
Lansdale Pennsylvania 19446 (US)

(74) Representative: Abitz, Walter, Dr.-Ing. et al
Abitz, Morf, Gritschneder P.O. Box 86 01 09
D-8000 München 86 (DE)

EP 0 024 348 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to new hypocholesterolemic and hypolipemic compounds having the structure (I)

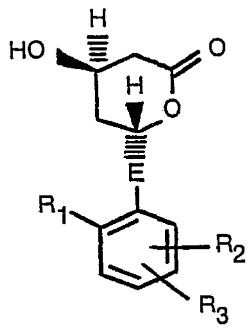

wherein

E is —CH=CH— or —$CH_2$—$CH_2$—

$R_1$ is phenyl substituted by halo and/or $C_{1-4}$ alkyl, or $OR_4$ in which $R_4$ is phenyl $C_{1-3}$ alkyl unsubstituted or substituted with halo,

$R_2$ and $R_3$ are independently hydrogen, halo, or $C_{1-4}$ alkyl and the corresponding dihydroxy acids resulting from the hydrolytic opening of the lactone ring, and the pharmaceutically acceptable salts of said acids, or the lower alkyl and phenyl, dimethylamino or acetylamino-substituted lower alkyl esters of said dihydroxy acids; all said compounds being the 4-*R* enantiomer of the *trans* racemate whose structure is shown above.

A preferred embodiment of this invention relates to those structures of general formula I wherein

E is —CH=CH—, and

$R_1$ is substituted phenyl in which the substituent is halo, and/or $C_{1-4}$ alkyl, or the corresponding dihydroxy acids resulting from the hydrolytic opening of the lactone ring or the pharmaceutically acceptable salts of said dihydroxy acids, or the lower alkyl and phenyl, dimethylamino or acetylamino-substituted lower alkyl esters of said dihydroxy acids; all said compounds being the enantiomer having a 4 *R* configuration in the tetrahydropyran moiety of the *trans* racemate shown in general formula I.

The designation 4 *R* with respect to these compounds indicates that the absolute configuration in space at the 4-carbon of the pyranone ring is believed to be the Rectus (R) series. All the compounds synthesized have been found to be dextro-rotatory.

Background of the Invention

It is known that certain mevalonate derivatives inhibit the biosynthesis of cholesterol, cf F.M. Singer, et al., *Proc. Soc. Exper. Biol. Med., 102,* 270 (1959) and F. H. Hulcher, *Arch. Biochem. Biophys., 146,* 422 (1971). Nevertheless, the activity of these known compounds has not always been found to be satisfactory, i.e. to have practical application.

Recently, Endo et al, reported (U.S. Letters Patent 4,049,495, Patent 4,137,322 and Patent 3,983,140) the production of a fermentation product which was quite active in the inhibition of cholesterol biosynthesis. This natural product, now called compactin, was reported by Brown et al., (*J. Chem. Soc. Perkin I,* 1165 (1976)) to have a complex mevalonolactone structure. However, the low rate of production from fermentation broths appears to limit the supply of this natural product.

A recent Belgian patent 867,421 disclosed a group of synthetic compounds of the generic formula II

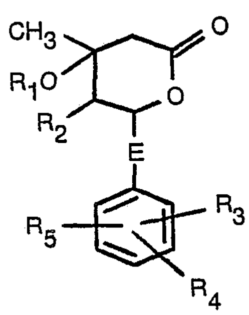

II

in which E represents a direct bond, a $C_{1-3}$ alkylene bridge or a vinylene bridge and the various R's represent a variety of substituents.

The activity reported in the Belgian patent is less than 1% that of compactin.

EP—A 10951 describes 4-hydroxy-3,4,5,6-tetrahydro-2H-pyran-2-ones which are substituted in the 6-position by —A—Z, A being an alkylene group and Z a substituted or unsubstituted aryl group. Alkyl groups

or halogen atoms are among the substituents which come under consideration for the aryl group. There is no pointer to optionally substituted phenyl groups also coming under consideration as substituents at the aryl group or that such compounds have a particularly favorable biological activity.

FR—A 2 392 016 describes 4-hydroxy-4-methyl-3,4,5,6-tetrahydro-2H-pyran-2-ones. Apart from the fact that R enantiomers are not mentioned in that reference, it is pointed out that all compounds of that reference have a methyl group in the 4-position. It has, however, been established that precisely compounds without that methyl group have a particularly favorable biological activity.

Liebigs Annalen Chem., 1979, 484—491, describes R and S enantiomers of 4-hydroxy-3,4,5,6-tetrahydro-2H-pyran-2-ones having a Ph—CH=CH substituent in 6-position, however, no substituents being provided for at the Ph (phenyl group).

The Present Invention

We have found that the enantiomers of the *trans* compounds of Formula I having a 4 *R* configuration in the tetrahydropyran moiety especially those having a 2,4,6-trisubstitution pattern on the aromatic ring are unexpectedly potent inhibitors of cholesterol biosynthesis, approaching and surpassing the order of magnitude of compactin.

The cited Belgian patent, shows no recognition of the stereochemistry of these compounds, let alone the fact that an unexpectedly large improvement in the activity would result from the separation of the *cis* and *trans* racemates and the latter's resolution, especially when the preferred 2,4,6-trisubstitution occurs in the phenyl ring. However, it has been found that the 4 *R* enantiomers of the *trans* racemates corresponding to formula I specifically inhibit with high potency the activity of 3-hydroxy-3-methylglutaryl coenzyme A reductase, which is known to be the rate limiting enzyme in the process of biosynthesis of cholesterol.

The inhibitory activity of these compounds for the biosynthesis of cholesterol has been measured by two methods. The experimental method A has been realized *in vitro* by the method of H. J. Knauss, et al., *J. Biol. Chem., 234,* 2835 (1959) and the activity was expressed as the molar concentration $IC_{50}(M)$ necessary for the inhibition of 50% of the enzymatic activity. The experimental method B was the method of A. A. Kandutsch, et al., *J. Biol. Chem., 248,* 8403 (1973) for measuring the quantity of $^{14}C$-cholesterol biosynthesis from acetic acid-$^{14}C$ in mouse L cells. The activity is expressed for inhibition of 50% of the biosynthesis of cholesterol.

The results obtained in these two assays, as reported in the cited Belgian patent, show $IC_{50}$ values of $10^{-4}$ to $10^{-6}$ in both tests. The smallest 50% effective dose cited is about $4 \times 10^{-6}$, whereas the value for compactin, in the same tests, is about $0.8 \times 10^{-8}$. We have found that the inhibitory potency is greatly increased by such isomer separation, especially when this is combined with optimal selection of a 2,4,6-arrangement of $R_1$, $R_2$ and $R_3$ in the phenyl ring (i.e. selection of $R_2$ and $R_3$ as 2,4-dichloro and $R_1$ as 6-phenyl or phenylalkoxy), especially with a halogen (preferably fluorine) on the 6-phenyl or phenylalkoxy group. Thus the (+) *trans* enantiomer of 6-[2-(2,4-dichloro-6-phenylmethoxyphenyl)ethyl]-3,4,5,6-tetrahydro-4-hydroxy-2H-pyran-2-one (Example 14) (a preferred compound of this invention) gives an $IC_{50}$ of $6.8 \times 10^{-8}$ in the test by method A. An even more potent and preferred compound of this invention, (+)-(E)-(3R*,5S*)-7-(3,5-dichloro-4'-fluoro[1,1'-biphenyl]-2-yl)-3,5-dihydroxy-6-heptenoic acid, ammonium salt (Example 16) gives an $IC_{50}$ of about $2.8 \times 10^{-9}$, a potency greater than that of compactin, the product described by Endo, when the two compounds are tested as the sodium salts of their corresponding hydroxy acid forms.

The preparation of the compounds of this invention is illustrated in the Flow Sheets.

Flow Sheet I shows the general scheme for synthesizing compounds with a vinylene bridge between the lactone and benzene rings. A starting benzaldehyde is converted to the corresponding cinnamaldehyde (this forms the bridging group) and this is subjected to an aldol reaction to elaborate a hydroxy keto ester from the terminal aldehydic moiety. Reduction of the hydroxy keto ester affords the dihydroxy ester which upon lactonization, gives the lactone. The lactone is then separated chromatographically into its *cis* and *trans* racemates and the latter racemate is resolved to give the desired 4 *R trans* enantiomer.

Flow Sheet II shows the further conversion of the 4 *R trans* lactones into the corresponding dihydroxy acids and their salts and esters. Although this sequence is shown with the —CH=CH— bridged compounds, the same sequence can be used to give the corresponding acids, salts and esters of any of the other bridged compounds.

Flow Sheet III shows the synthetic routes for the preparation of the ethylene bridging group, represented by E in formula I. Compounds with an ethylene ($—CH_2—CH_2—$) bridge between the rings are prepared by reduction of the vinylene bridged compounds prepared in Flow Sheet I. Separation of the *cis* and *trans* racemic lactones and the subsequent resolution of the *trans* racemate to obtain the 4 *R* enantiomer are carried out as described in Flow Sheet I.

## FLOW SHEET I

Synthesis of Vinylene Bridged Compounds

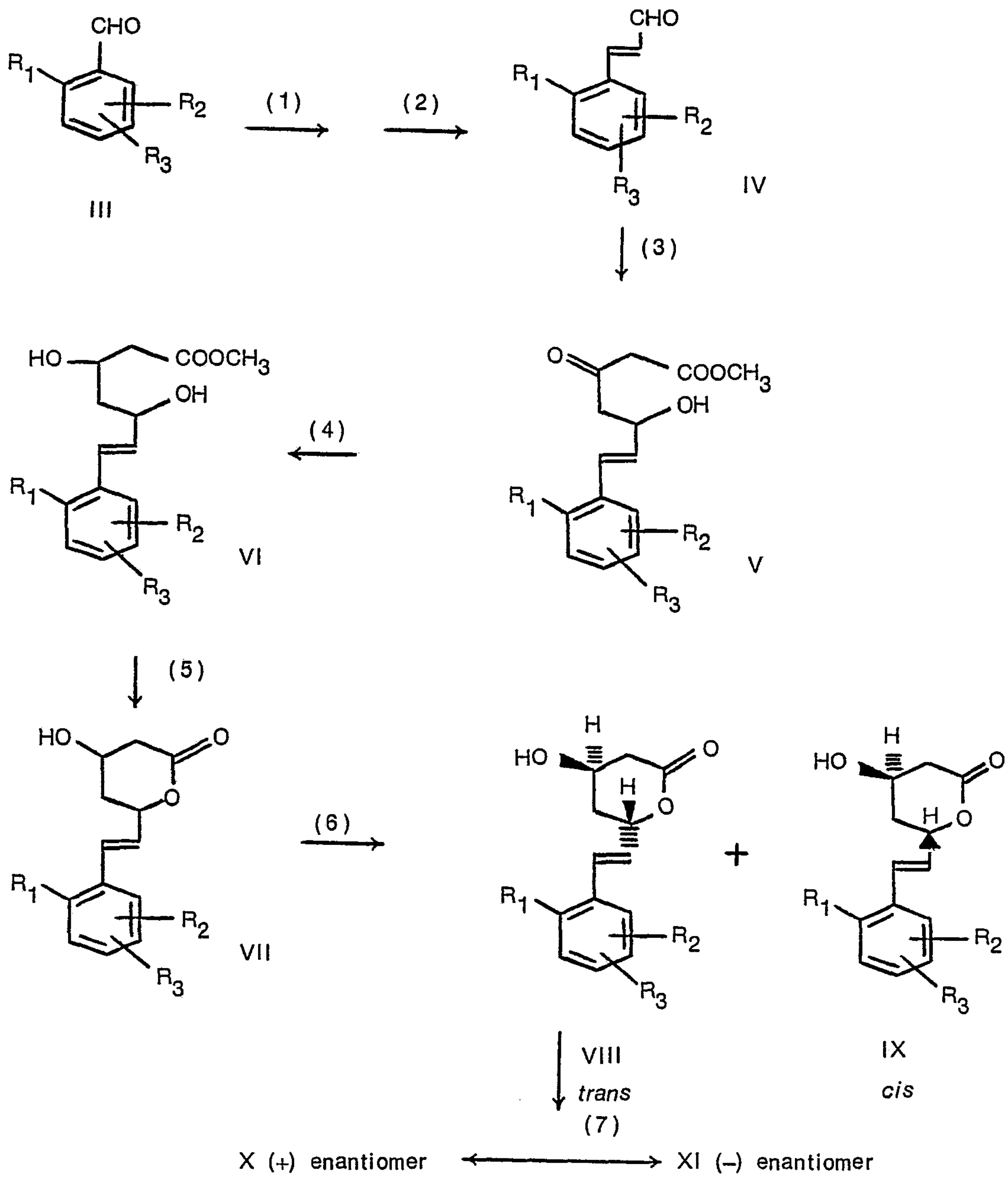


III

IV

V

VI

VII

VIII *trans*

IX *cis*

X (+) enantiomer

XI (–) enantiomer

DEFINITIONS: $R_1$, $R_2$ and $R_3$ are as defined in specifications for Formula I.

FLOW SHEET II

Preparation of Salts, Esters, Free Dihydroxy Acids

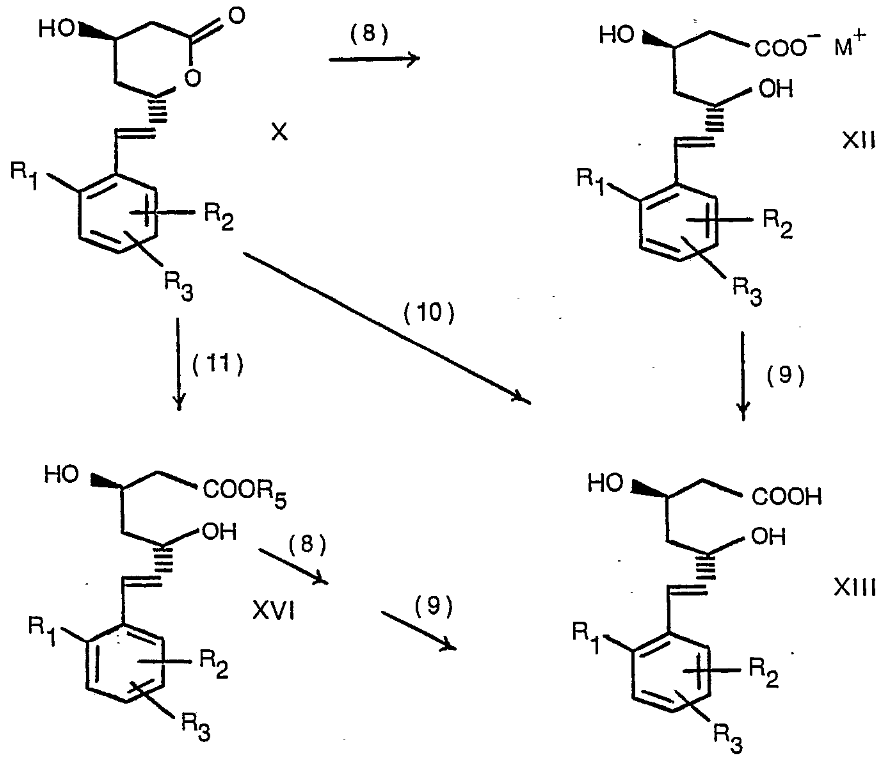


X

XII

XVI

XIII

DEFINITIONS:

$R_1$, $R_2$ and $R_3$ are as defined in the specifications for Formula I

$R_5$ = $C_{1-5}$ lower alkyl or $C_{1-5}$ lower alkyl substituted by a phenyl, dimethylamino or acetamino group

$M^+$ = a pharmaceutically acceptable cation.

FLOW SHEET III

COMPOUNDS WITH ETHYLENE BRIDGING GROUPS

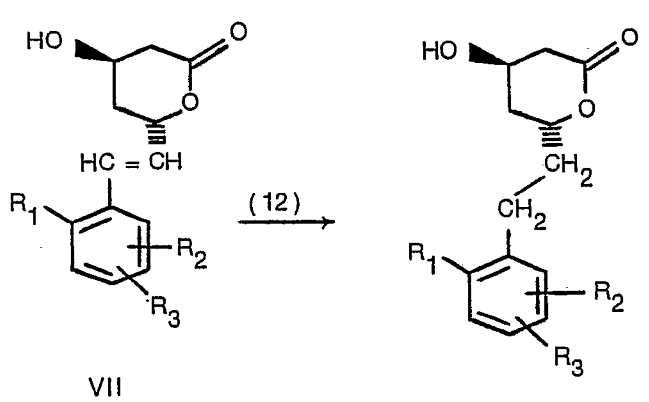


XXII

VII

Reactions in Flow Sheets I—III

1. When $R_1$, $R_2$ or $R_3$ is HO— or bears a hydroxyl substituent, the HO— group is etherified using a reagent $R_4X$ in a suitable solvent such as DMF and the like in the presence of a suitable base, preferably an alkali metal carbonate such as $K_2CO_3$, to give the corresponding ether $R_4O$— which can be carried through the remainder of the synthesis. If it is desired to remove $R_4$ at a later synthetic step, $R_4$ is chosen as an easily removable group such as $CH_3OCH_2CH_2OCH_2$— (the MEM protecting group). The MEM group is removed readily by treatment with a Lewis acid catalyst such as $ZnBr_2$ in a suitable solvent such as $CH_2Cl_2$ and the like. When the starting material is devoid of a hydroxyl group, step (1) is omitted.

2. Aldol Reaction. This can be run in several ways:

a) The classical Aldol synthesis in which acetaldehyde is condensed with the starting benzaldehyde, the resulting β-hydroxyaldehyde is acetylated with acetic anhydride and acetic acid is eliminated thermally to give the corresponding cinnamaldehyde.

b) The directed Aldol condensation in which the anion of an appropriately N-substituted ethylidenamine, such as ethylidenecyclohexylamine and the like, is condensed with the starting benzaldehyde at or below room temperature in an aprotic solvent, such as THF and the like, to afford a β-hydroxy-β-phenyl-propylidenylamine which, upon concomitant dehydration and imine hydrolysis in an acidic medium, such as dilute aqueous HCl, provides the corresponding cinnamaldehyde.

c) The use of a nucleophilic acetaldehyde equivalent in which *cis*-2-ethoxyvinyllithium, generated from *cis*-1-ethoxy-2-tri-*n*-butylstannylethylene, is condensed with the starting benzaldehyde to give an allylic alcohol which is subsequently rearranged, under suitable acidic conditions, to the corresponding cinnamaldehyde.

3. Dianion Step. Reaction with the dianion of acetoacetic ester in a suitable aprotic solvent such as THF, dioxane and the like.

4. Reduction with $NaBH_4$ in a suitable solvent such as methanol, ethanol and the like at or below room temperature.

5. Lactonization. Saponification by base (e.g. NaOH) in aqueous alcohol followed by acidification and cyclodehydration by heating in toluene. NOTE: Steps 3, 4 and 5 are usually carried out sequentially without purification of compounds V and VI.

6. Separation of the *cis* and *trans* racemic mixtures by chromatography on silica gel or crystallization.

7. Resolution of the *trans* racemate into its enantiomers by treating the (±)-*trans* lactone with either *d*-(+) or *1*-(−)-α-methylbenzylamine to give the diastereomeric dihydroxy amides which are separated by chromatography or crystallization. Hydrolysis of each pure diastereomeric amide under basic conditions, such as ethanolic NaOH and the like, affords the corresponding enantiomerically pure dihydroxy acid which, upon lactonization, e.g., in refluxing toluene, provides the pure (+)-*trans* or (−)-*trans* enantiomer. Stereochemistry depends on the absolute stereochemistry of the diastereomeric amide from which it is derived.

8. Saponification with $M^{+-}OH$ where $M^+$ is an alkali metal cation.

9. Careful acidification.

10. Mild hydrolysis

11. Nucleophilic opening of the lactone ring with an alochol, $R_5OH$, in the presence either of a basic catalyst, particularly the corresponding alkoxide, $R_5O^-$, or an acidic catalyst such as an acidic ion exchange resin, e.g. *Amberlite 120.*

12. Hydrogenation in the presence of a suitable catalyst such as Rhodium or Palladium on carbon.

A further aspect of the present invention is a pharmaceutical composition consisting of at least one of the compounds of formula I in association with a pharmaceutical vehicle or diluent. The pharmaceutical composition can be formulated in a classical manner utilizing solid or liquid vehicles or diluents and pharmaceutical additives of a type appropriate to the mode of desired administration. The compounds can be administered by an oral route, for example, in the form of tablets, capsules, granules or powders, or they can be administered by a parenteral route in the form of injectable preparations. The dose to be administered depends on the unitary dose, the symptoms, and the age and the body weight of the patient. A dose for adults is preferably between 200 and 2,000 mg per day, which can be administered in a single dose or in the form of individual doses from 1—4 times per day.

A typical capsule for oral administration contains active ingredient (250 mg), lactose (75 mg) and magnesium stearate (15 mg). The mixture is passed through a 60 mesh sieve and packed into a No. 1 gelatin capsule.

A typical injectible preparation is produced by asceptically placing 250 mg of sterile active ingredient into a vial, asceptically freeze-drying and sealing. For use, the contents of the vial are mixed with 2 ml of physiological saline, to produce an injectible preparation.

The compounds of this invention also have useful antifungal activities. For example, they may be used to control strains of *Penicillium sp., Aspergillus niger, Cladosporium sp., Cochliobolus miyabeorus* and *Hilminthosporium cynodnotis.* For those utilities they are admixed with suitable formulating agents, powders, emulsifying agents or solvents such as aqueous ethanol and sprayed or dusted on the plants to be protected.

This invention can be illustrated by the following examples in which ratios of solvents are in volumes and percentages, unless otherwise indicated, or by weight.

Example 1

Preparation of (E)-6-[2-(2,4-Dichloro-6-phenylmethoxyphenyl)ethenyl]-3,4,5,6-tetrahydro-4-hydroxy-2H-pyran-2-one

*Step A.* Preparation of 2,4-Dichloro-6-phenylmethoxybenzaldehyde

Potassium carbonate (9.4 g, 67.8 mmole) was added to a stirred solution of 4,6-dichlorosalicylaldehyde (10.8 g, 56.5 mmole) in dimethylformamide (80 ml). The resulting mixture was stirred at 60°C for 30 minutes and treated with benzyl bromide (10.6 g, 62.1 mmole). This mixture was stirred one hour at 60°C and then poured into ice water (1000 ml) to give the title compound (15.9 g, 100%) which melted at 98—100°C after recrystallization from hexane. pmr ($CDCl_3$) δ 5.10 (2H, s), 7.33 (5H, s), 10.40 (H, s).

Analysis Calc. for $C_{14}H_{10}Cl_2O_2$
Calc.: C, 59.81; H, 3.58
Found: C, 59.98; H, 3.58.

*Step B.* Preparation of (E)-2,4-Dichloro-6-phenylmethoxycinnamaldehyde

A stirred suspension of 2,4-dichloro-6-phenyl-methoxybenzaldehyde (15.5 g, 55.1 mmole) in acetaldehyde (30 ml) was cooled to 5°C and treated with 25% methanolic potassium hydroxide (1.4 ml, 6.24 mmole) at such a rate that the internal temperature was maintained at 25—30°C. The resulting solution was stirred for 30 minutes in the ice bath, treated with acetic anhydride (30 ml) and then heated at 100°C for 30 minutes. After cooling to 30°C the solution was treated with water (84 ml) and 12 N hydrochloric acid (7 ml). The resulting mixture was refluxed for 30 minutes and then cooled in an ice bath to give a gummy solid which was recrystallized from cyclohexane to give the title compound (5.6 g, 33%), mp 109—112°C: pmr ($CDCl_3$) δ 5.10 (2H, s), 7.33 (5H, s), 9.68 (H, d).

Analysis Calc. for $C_{16}H_{12}Cl_2O_2$
Calc.: C, 62.56; H, 3.94
Found: C, 62.66; H, 3.98

*Alternate Step B.* Preparation of (E)-2,4-Dichloro-6-phenylmethoxycinnamaldehyde

A 1.6 M solution (18.8 ml, 30 mmole) of *n*-butyllithium in hexane was added cautiously to a stirred solution of freshly distilled diisopropylamine (3.0 g, 30 mmole) in anhydrous tetrahydrofuran (200 ml) maintained at 0°C under a nitrogen atmosphere. The resulting solution was stirred at 0°C for 15 minutes and then treated with ethylidenecyclohexylamine (3.75 g, 30 mmole). The solution was stirred 15 minutes at 0°C, cooled to −78°C and treated with a solution of 2,4-dichloro-6-phenylmethoxybenzaldehyde (8.4 g, 30 mmole) in anhydrous tetrahydrofuran (50 ml). The resulting red solution was stirred at −78°C for 15 minutes and then at 25°C for 60 minutes. The reaction solution was treated with water (200 ml) and extracted with ether (3 × 200 ml). The organic extracts were combined, washed with brine (3 × 100 ml), dried over magnesium sulfate and filtered. The filtrate was evaporated *in vacuo*, leaving the desired intermediate hydroxyimine as a brown viscous oil (12.5 g): pmr ($CDCl_3$) δ 5.10 (2H, s), 5.50 (H, t), 7.37 (5H, s), 7.70 (H, s).

A solution of the oily imine (12.5 g) in tetrahydrofuran (110 ml) was treated with a solution of oxalic acid dihydrate (11 g, 87.2 mmole) in water (22 ml). The resulting solution was refluxed for 30 minutes, cooled to 25°C and poured into water (500 ml). The resulting mixture was extracted with ether (3 × 200 ml). The organic extracts were combined, washed with brine (3 × 50 ml), dried over magnesium sulfate and filtered. The filtrate was evaporated *in vacuo*, leaving the title compound as a tan solid. The title compound was purified by recrystallization from cyclohexane to give yellow needles (4.7 g, 51%) melting at 109—111°C: pmr ($CDCl_3$) δ 5.11 (2H, s), 7.33 (5H, s), 9.68 (H, d).

*Alternate to Alternate Step B.* Preparation of (E)-2,4-Dichloro-6-phenylmethoxycinnamaldehyde

A 1.37 M solution (24.1 ml, 33 mmole) of *n*-butyllithium in hexane was added cautiously to a stirred solution of *cis*-1-ethoxy-2-tri-*n*-butylstannylethylene (11.9 g, 33 mmole) in anhydrous tetrahydrofuran (75 ml) maintained at −78°C under a nitrogen atmosphere. The resulting solution was stirred at −78°C for one hour and then treated with a solution of 2,4-dichloro-6-phenylmethoxybenzaldehyde (8.4 g, 30 mmole) in anhydrous tetrahydrofuran (50 ml). The resulting brown solution was stirred at −78°C for one hour and then allowed to warm to 20°C. The reaction solution was quenched with saturated aqueous sodium bicarbonate (25 ml), diluted with water (100 ml) and then extracted with ether (2 × 200 ml). The organic extracts were combined, washed with brine (2 × 100 ml), dried over magnesium sulfate and filtered. The filtrate was evaporated *in vacuo*, leaving the desired intermediate allylic alcohol as a yellow oil.

The oil was chromatographed on a silica column (400 g) to cause allylic rearrangement to the desired product. Elution with methylene chloride (200 ml) provided a forerun containing tetrabutyltin which was discarded. Continued elution with methylene chloride/methanol (98:2, V:V; 1500 ml) gave the title compound as a pale yellow solid, mp 109—111°C (6.4 g, 70%).

*Step C.* Preparation of Methyl (E)-7-(2,4-dichloro-6-phenylmethoxyphenyl)-5-hydroxy-3-oxo-6-heptenoate

Methyl acetoacetate (9.56 g, 82.3 mmole) was added dropwise to a stirred suspension of sodium hydride (50% oil suspension) (3.95 g, 82.3 mmole) in anhydrous tetrahydrofuran at 0°C under a nitrogen atmosphere. The resulting solution was stirred 15 minutes at 0°C and then treated with a 1.6 M solution (51.5 ml, 82.3 mmole) of *n*-butyllithium in hexane over 5 minutes. The resulting yellow solution was stirred 15 minutes at 0°C and then treated with a solution of (E)-2,4-dichloro-6-phenylmethoxycinnamaldehyde (25.3 g, 82.3 mmole) in anhydrous tetrahydrofuran (150 ml). The resulting orange solution was stirred 15 minutes at 0°C and then quenched by dropwise addition of 12 N hydrochloric acid (ca. 20 ml). The reaction mixture was diluted with water (100 ml) and extracted with ether (3 × 300 ml). The organic extracts were combined, washed with brine (3 × 100 ml), dried over magnesium sulfate and filtered. The filtrate was evaporated *in vacuo* leaving the title compound as a yellow oil (34.8 g, 100%): pmr ($CDCl_3$) δ 2.75 (2H, d), 3.45 (2H, s), 3.72 (3H, s), 4.71 (H, m), 5.50 (2H, s), 7.37 (5H, s).

*Step D.* Preparation of Methyl (E)-7-(2,4-Dichloro-6-phenylmethoxyphenyl)-3,5-dihydroxy-6-heptenoate

Sodium tetrahydridoborate (1.55 g, 41.1 mmole) was added with stirring to a cooled solution (5°C) of methyl (E)-7-(2,4-dichloro-6-phenylmethoxyphenyl)-5-hydroxy-3-oxo-6-heptenoate (34.8 g, 82.3 mmole) in ethanol (200 ml) at a rate sufficient to maintain the internal temperature at 15—20°C. The resulting solution was stirred with ice-bath cooling for 15 min. and then acidified with 6N hydrochloric acid. The resulting mixture was diluted with water (500 ml) and extracted with ether (3 × 250 ml). The organic extracts were combined, washed with brine (4 × 100 ml), dried over magnesium sulfate and filtered. The filtrate was evaporated *in vacuo*, leaving the title compound as a yellow oil (34.8 g, 99.5%): pmr $CDCl_3$) δ 2.45 (2H, d), 3.65 (3H, s), 4.18 (H, m), 4.45 (H, m), 4.98 (2H, s), 7.28 (5H, s).

*Step E.* Preparation of (E)-7-(2,4-Dichloro-6-phenylmethoxyphenyl)-3,5-dihydroxy-6-heptenoic acid

A solution of methyl (E)-7-(2,4-dichloro-6-phenylmethoxyphenyl)-3,5-dihydroxy-6-heptenoate (34.8 g, 81.8 mmole), 1 N sodium hydroxide (82 ml, 82 mmole) and ethanol (200 ml) was stirred at 25°C for 15 min. The reaction solution was acidified with 6N hydrochloric acid, diluted with water (400 ml) and extracted with ether (3 × 200 ml). The combined organic extracts were washed with brine (3 × 100 ml), dried over magnesium sulfate and filtered. The filtrate was evaporated *in vacuo*, leaving the title compound as an orange oil (33.3 g, 99%): pmr ($CDCl_3$) δ 2.47 (2H, d), 4.30 (2H, br m), 4.98 (2H, s), 7.30 (5H, s).

*Step F.* Preparation of (E)-6-[2-(2,4-Dichloro-6-phenylmethoxyphenyl)ethenyl]-3,4,5,6-tetrahydro-4-hydroxy-2H-pyran-2-one

A solution of (E)-7-(2,4-dichlorophenylmethoxyphenyl)-3,5-dihydroxy-6-heptenoic acid (33.3 g, 81.3 mmole) in toluene (300 ml) was heated at reflux in a Dean-Stark apparatus. After 2 hours the Dean-Stark apparatus was replaced with a soxhlet containing 3Å molecular sieves (100 g). The solution was refluxed for an additional 4 hours and then the toluene was removed *in vacuo* leaving a yellow oil (31.7 g) which is a mixture of *cis* and *trans* isomers of the title compound. The oil was chromatographed on a silica gel column (900 g). Elution with methylene chloride-acetone (9:1, V:V; 4000 ml) provided a forerun which was discarded. Continued elution with the same eluant (500 ml) gave the *trans* isomer of the title compound as a pale yellow solid (5.8 g).

Further elution of the column with the same eluant (3250 ml) gave a tan solid (8.8 g), which is a mixture of the *cis* and *trans* isomers of the title compound. This *cis/trans* mixture was chromatographed using a Waters Prep LC500. Separation of this mixture was accomplished by using two prep PAK-500/silica cartridges in series and eluting with methylene chloride-acetone (9:1, V:V). Using the shave recycle technique, the *cis* (4.7 g) and the *trans* (3.3 g) isomers of the title compound were obtained. The fractions of the *trans* isomer, collected from the two chromatographys, were combined and recrystallized from *n*-butyl chloride to give the *trans* isomer of the title compound (7.3 g, 23%), mp 130—131°C: pmr ($CDCl_3$) δ 2.64 (2H, m), 4.30 (H, m), 5.07 (2H, s), 5.30 (H, m), 7.42 (5H, s).

Analysis Calc. for $C_{20}H_{18}Cl_2O_4$
Calc.: C, 61.08; H, 4.61
Found: C, 61.12; H, 4.60.

The *cis* isomer (4.3 g, 13%) of the title compound melted at 130—131.5°C after recrystallization from *n*-butyl chloride: pmr ($CDCl_3$) δ 4.30 (H, m), 4.83 (H, m), 5.12 (2H, s), 7.47 (5H, s).

Analysis Calc. for $C_{20}H_{18}Cl_2O_4$
Calc.: C, 61.08; H, 4.61
Found: C, 61.55; H, 4.63.

## Example 2

Starting with 4,6-dichlorosalicylaldehyde but substituting equimolar amounts of the following alkyl halide or tosylate in place of benzyl bromide in Step A of Example 1 and following the procedure of Steps A through F there was obtained a corresponding amount of the appropriate end product listed below.

| Alkyl Halide or Tosylate | End Product | Isomer | M.P. °C | | Calc. | Fd. |
|---|---|---|---|---|---|---|
| 3,4-dichlorobenzyl chloride | (E)-6-{2-[2,4-dichloro-6-(3,4-dichlorophenyl-methoxy)phenyl]ethenyl}-3,4,5,6-tetrahydro-4-hydroxy-2H-pyran-2-one | trans | 73–75 | C<br>H | 52.27<br>3.73 | 52.03<br>3.74 |
| 4-chlorobenzyl bromide | (E)-6-{2-[6-(4-chloro-phenylmethoxy-2,4-dichlorophenyl]-ethenyl}-3,4,5,6-tetrahydro-4-hydroxy-2H-pyran-2-one | trans | 111.5–113 | C<br>H | 56.16<br>4.01 | 56.08<br>3.98 |
| 1-bromo-3-phenyl-propane | (E)-6-{2-[2,4-dichloro-6-(3-phenylpropoxy)phenyl]-ethenyl}-3,4,5,6-tetrahydro-4-hydroxy-2H-pyran-2-one | trans | 93–94 | C<br>H | 62.71<br>5.26 | 62.66<br>5.25 |
| 4-fluorobenzyl bromide | (E)-6-2-[2,4-dichloro-6-(4-fluorophenyl-methoxy)phenyl]ethenyl-3,4,5,6-tetrahydro-4-hydroxy-2H-pyran-2-one | trans | 124–6 | C<br>H | 58.41<br>4.17 | 58.26<br>4.20 |
| | | cis | 131–3 | C<br>H | 58.41<br>4.17 | 58.29<br>4.06 |
| 2-fluorobenzyl chloride | (E)-6-2-[2,4-dichloro-6-(2-fluorophenylmethoxy)-phenyl]ethenyl-3,4,5,6-tetrahydro-4-hydroxy-2H-pyran-2-one | trans | 140–141.5 | C<br>H | 58.41<br>4.17 | 58.43<br>4.20 |
| 2,4-difluorobenzyl bromide | (E)-6-{2-[2,4-dichloro-6-(2,4-difluorophenylmethoxy)-phenyl]ethenyl}-3,4,5,6-tetrahydro-4-hydroxy-2H-pyran-2-one | trans | 138–139 | C<br>H | 55.96<br>3.76 | 56.00<br>3.82 |

| Alkyl Halide or Tosylate | End Product | Isomer | M.P. °C | | Calc. | Fd. |
|---|---|---|---|---|---|---|
| 3-fluorobenzyl bromide | (E)-6-{2-[2,4-dichloro-6(3)-fluorophenylmethoxy)-phenyl]ethenyl}-3,4,5,6-tetrahydro-4-hydroxy-2H-pyran-2-one | trans | 91.5—92 | C<br>H | 58.41<br>4.17 | 58.48<br>4.22 |
| 2,3,4,5,6-penta-fluorobenzyl-chloride | (E)-6-{2-[2,4-dichloro-6-(2,3,4,5,6-pentafluoro-phenylmethoxy)phenyl]-ethenyl}-3,4,5,6-tetrahydro-4-hydroxy-2H-pyran-2-one 0.5 chlorobutane | trans | 72–75 | C<br>H | 49.90<br>3.33 | 49.87<br>3.35 |

## Example 3

Preparation of *Trans*-6-[2-(2,4-Dichloro-6-phenylmethoxyphenyl)ethyl]-3,4,5,6-tetrahydro-4-hydroxy-2H-pyran-2-one

A solution of *trans*-(E)-6-[2-(2,4-dichloro-6-phenylmethoxyphenyl)ethenyl]-3,4,5,6-tetrahydro-4-hydroxy-2H-pyran-2-one (1.1 g, 28 mmole) in tetrahydrofuran (50 ml) was magnetically stirred and hydrogenated at room temperature and atmospheric pressure in the presence of 110 mg of 5% rhodium on carbon catalyst until 1.5 molar equivalents of hydrogen had been consumed. After removing the catalyst by filtration, the filtrate was evaporated *in vacuo* leaving the title compound as a pale yellow oil. The oil was chromatographed on a silica gel column (200 g). Elution with acetone-methylene chloride (1:9, V:V; 560 ml) provided a forerun which was discarded. Continued elution with the same eluant (240 ml) gave the title compound as a colourless oil which solidified when it was triturated with ether. The title compound was purified by recrystallization from ether-hexane (1:1, V:V; 20 ml) to give colorless needles (0.67 g, 61%) melting at 99—101°C: pmr ($CDCl_3$) δ 1.83 (4H, m), 2.60 (2H, m), 2.90 (2H, m), 4.30 (H, m), 4.62 (H, m), 5.05 (2H, s), 7.42 (5H, s).

Analysis Calc. for $C_{20}H_{20}Cl_2O_4$

| | | |
|---|---|---|
| Calc.: | C, 60.77; | H, 5.10 |
| Found: | C, 60.96; | H, 4.85. |

## Example 4

By substituting an equimolar amount of the following 2H - pyran - 2 - one for *trans* - (E) - 6 - [2 - (2,4 - dichloro - 6 - phenylmethoxyphenyl)ethenyl] - 3,4,5,6 - tetrahydro - 4 - hydroxy - 2H - pyran - 2 - one in Example 3 and following the procedure of Example 3, there was obtained a corresponding amount of the following end products.

| 2H-Pyran-2-one | End Product | M.P. °C. | | Calc. | Fd. |
|---|---|---|---|---|---|
| *trans*-(E)-6-{2-[2,4 dichloro-6-(4-fluoro-phenylmethoxy)phenyl]-ethenyl}-3,4,5,6-tetra-hydro-4-hydroxy | *trans*-6-{2-[2,4-dichloro-6-(4-fluorophenylmethoxy)-phenyl]ethyl}-3,4,5,6-tetrahydro-4-hydroxy-2H-pyran-2-one | 151—152 | C<br>H | 58.12<br>4.63 | 58.13<br>4.68 |
| *trans*-(E)-6-{2-[2,4-dichloro-6-(2-fluoro-phenylmethoxy)phenyl]-ethenyl}-3,4,5,6-tetra-hydro-4-hydroxy | *trans*-6-{2-[2,4-dichloro-6-(2-fluorophenylmethoxy)-phenyl]ethyl}-3,4,5,6-tetrahydro-4-hydroxy-2H-pyran-2-one | 98—100 | C<br>H | 58.12<br>4.63 | 58.24<br>4.63 |
| *trans*-(E)-6-{2-[2,4-dichloro-6-(3-fluoro-phenylmethoxy)phenyl]-ethenyl}-3,4,5,6-tetra-hydro-4-hydroxy | *trans*-6-{2-[2,4-dichloro-6-(3-fluorophenylmethoxy)-phenyl]ethyl}-3,4,5,6-tetrahydro-4-hydroxy-2H-pyran-2-one | 106—115 | C<br>H | 58.12<br>4.63 | 58.20<br>4.72 |

Example 5

Resolution of the Optical Isomers of (±) *Trans*-6-[2-2,4-dichloro-6-phenylmethoxyphenyl)ethyl]-3,4,5,6-tetrahydro-4-hydroxy-2H-pyran-2-one

*Step A.* Preparation and Separation of Diastereomeric Amides (Diastereomers A and B)

By substituting an equimolar amount of (±) *trans* - 6 - [2 - (2,4 - dichloro - 6 - phenylmethoxyphenyl)ethyl] - 3,4,5,6 - tetrahydro - 4 - hydroxy - 2H - pyran - 2 - one for (±) *trans* - (E) - 6 - [2 - (2,4-dichlorophenyl)ethenyl] - 3,4,5,6-tetrahydro - 4 - hydroxy - 2H - pyran - 2 - one and replacing *d* - (+) - α - methylbenzylamine with the *1* - (−) - isomer in Step A of the following Example 5A and following the procedure described in Step A of Example 5A, there were obtained a corresponding amount of the diastereomeric amides:

*Diastereomer A:* mg 177—179°C; pmr ($CDCl_3$) δ 1.45 (3H, d), 2.22 (2H, d), 2.83 (2H, m), 3.74 (H, m), 4.13 (H, m), 5.04 (2H, s), 6.86 (H, d), 7.05 (H, d), 7.33 (5H, s), 7.82 (5H, s)

Analysis Calc. for $C_{28}H_{31}Cl_2NO_4$

| | | | |
|---|---|---|---|
| Calc.: | C, 65.11; | H, 6.05; | N, 2.71 |
| Found: | C, 65.28; | H, 6.34; | N, 2.95 |

*Diastereomer B:* mp 130—132°C; pmr ($CDCl_3$) δ 1.45 (3H, d), 2.22 (2H, d), 2.83 (2H, m), 3.74 (H, m), 4.13 (H, m), 5.04 (2H, s), 6.86 (H, d), 7.05 (H, d), 7.33 (5H, s), 7.82 (5H, s).

Analysis Calc. for $C_{28}H_{31}Cl_2NO_4$

| | | | |
|---|---|---|---|
| Calc.: | C, 65.11; | H, 6.05; | N, 2.71 |
| Found: | C, 65.24; | H, 6.27; | N, 2.88 |

*Step B.* Preparation of (+)-*Trans*-6-[2-(2,4-dichloro-6-phenylmethoxyphenyl)ethyl]-3,4,5,6-tetrahydro-4-hydroxy-2H-pyran-2-one. 0.1 *n*-Butyl Chloride Solvate

By substituting an equimolar amount of diastereomer A from the preceeding step for diastereomer A in Step B of Example 5A and following the procedure described therein, there was obtained a corresponding amount of the title compound, mp 108—112°C; pmr ($CDCl_3$) δ 1.91 (4H, m), 2.61 (2H, m), 2.93 (2H, m), 4.30 (H, m), 4.70 (H, m), 5.06 (2H, s), 6.33 (H, d), 7.02 (H, d), 7.43 (5H, s).

Analysis Calc. for $C_{20}H_{20}Cl_2O_4.0.1C_4H_9Cl$

| | | |
|---|---|---|
| Calc.: | C, 60.56; | H, 5.21 |
| Found: | C, 60.93; | H, 5.73 |

$[\alpha]_D^{25} = +16.0°$ (c, 0.1; chloroform)

*Step C.* Preparation of (−)-*Trans*-6-[2-(2,4-dichloro-6-phenylmethoxyphenyl)ethyl]-3,4,5,6-tetrahydro-4-hydroxy-2H-pyran-2-one.0.1 *n*-Butyl chloride Solvate

By substituting an equimolar amount of diastereomer B from Step A above for diastereomer B in Step C of Example 5A and following the procedure described therein, there was obtained a corresponding amount of the title compound, mp 104—111°C; pmr ($CDCl_3$) δ 1.91 (4H, m), 2.61 (2H, m), 2.93 (2H, m), 4.30 (H, m), 4.70 (H, m), 5.06 (2H, s), 6.33 (H, d), 7.02 (H, d), 7.43 (5H, s).

Analysis Calc. for $C_{20}H_{20}Cl_2O_4.0.1\ C_4H_9Cl$

| | | |
|---|---|---|
| Calc.: | C, 60.56; | H, 5.21 |
| Found: | C, 60.62; | H, 5.46 |

$[\alpha]_D^{25} = -17.7°$ (c, 0.1; chloroform).

Example 5A

This example only illustrates the details of the resolution method used for obtaining the compounds described in Example 5. The compounds described therein are not claimed.

Resolution of the Optical Isomers of (±)Trans-(E)-6-[2-(2,4-dichlorophenyl)ethenyl]-3,4,5,6-tetrahydro-4-hydroxy-2H-pyran-2-one

*Step A.* Preparation and Separation of Diastereomeric Amides (Diastereomers A and B)

A solution of (±)-*trans*-(E)-6-[2-(2,4-dichlorophenyl)ethenyl]-3,4,5,6-tetrahydro-4-hydroxy-2H-pyran-2-one (2.87 g, 10 mmole) in *d*-(+)-α-methylbenzylamine (15 ml) was stirred for 18 hours at 25°C and then

poured into water (100 ml). This aqueous mixture was acidified with 6N hydrochloric acid and then extracted with ether (3 × 100 ml). The ether extracts were combined, washed with brine (4 × 75 ml), dried over magnesium sulfate and filtered. Evaporation of the filtrate *in vacuo* gave the intermediate diastereomeric amides as a tan viscous oil (4.1 g).

The tan viscous oil (3.1 g, 7.6 mmole) was chromatographed on a silica gel column (200 g). Elution with acetone-methylene-chloride (1:4, V:V; 1200 ml) gave a forerun which was descarded. Continued elution with the same eluant (1000 ml) gave the diastereomeric amides as a viscous oil (3.0 g).

The diastereomeric amides were separated by chromatography on a Waters Prep LC500. The separation was accomplished by using two prep PAK—500 silica cartridges in series and eluting with acetonemethylene chloride (1:4, V:V). Using the shaverecycle technique, diastereomer A (1.36 g) and diastereomer B (1.20 g) were obtained.

Recrystallization of diastereomer A from *n*-butyl chloride gave colorless clusters (1.0 g) which melted at 106—108°C: pmr ($CDCl_3$) δ 1.47 (3H, d), 2.33 (2H, d), 4.30 (H, m), 5.17 (H, q), 7.33 (8H, m).

Analysis Calc. for $C_{21}H_{23}Cl_2NO_3$

| | | | |
|---|---|---|---|
| Calc.: | C, 61.77; | H, 5.68; | N, 3.43 |
| Found: | C, 61.78; | H, 5.78; | N, 3.50. |

Recrystallization of diastereomer B from *n*-butyl chloride-petroleum ether gave a pale yellow solid which melted at 55—60°C: pmr ($CDCl_3$) δ 1.47 (3H, d), 2.33 (2H, d), 4.30 (H, m), 5.17 (H, q), 7.33 (8H, m).

Analysis Calc. for $C_{21}H_{23}Cl_2NO_3$.

| | | | |
|---|---|---|---|
| Calc.: | C, 61.77; | H, 5.68; | N, 3.43 |
| Found: | C, 61.41; | H, 5.87; | N, 3.30. |

*Step B.* Preparation of (+)-*Trans*-(E)-6-[2-(2,4-dichlorophenyl)ethenyl]-3,4,5,6-tetrahydro-4-hydroxy-2H-pyran-2-one

Diastereomer A (0.74 g, 1.8 mmole) of Step A was dissolved in 95% ethanol (25 ml) containing 1N sodium hydroxide (3.6 ml, 3.6 mmole) and the solution was refluxed for 54 hours. The solvent was removed *in vacuo* and the residue was suspended in water (100 ml) and acidified with 6N hydrochloric acid. This aqueous mixture was extracted with ether (3 × 75 ml). The ether extracts were combined, washed with brine (2 × 50 ml), dried over magnesium sulfate and filtered. The filtrate was evaporated *in vacuo* leaving the intermediate acid as a yellow oil (0.54 g).

A solution of the yellow oil in toluene (150 ml) was refluxed through a soxhlet containing molecular sieves (3AÅ) for 5 hours. The solution was evaporated *in vacuo* leaving the title compound as a yellow solid. The title compound was purified by recrystallization from ether and then *n*-butyl chloride to give white needles (0.11 g, 20%) melting at 114—115°C, pmr ($CDCl_3$) δ 2.03 (2H, m), 2.73 (2H, m), 4.46 (H, m), 5.41 (H, m), 6.19 (H, dd) 7.01 (H, d), 7.14—7.50 (3H, m).

Analysis Calc. for $C_{13}H_{12}Cl_2O_3$

| | | |
|---|---|---|
| Calc.: | C, 54.37; | H, 4.21 |
| Found: | C, 54.51; | H, 4.32. |

$[\alpha]_D^{25} = +5.9°$ (c 0.425; chloroform)

*Step C.* Preparation of (−)*Trans*-(E)-6-[2-(2,4-Dichlorophenyl)ethenyl]-3,4,5,6-tetrahydro-4-hydroxy-2H-pyran-2-one

Diastereomer B (1.1 g, 2.7 mmole) of Step A was dissolved in 95% ethanol (25 ml) containing 1N sodium hydroxide (5.4 ml, 5.4 mmole) and the solution was refluxed for 18 hours. The ethanol was removed *in vacuo* and the residue was suspended in water (100 ml) and acidified with 6N hydrochloric acid. This aqueous mixture was extracted with ether (2 × 100 ml). The ether extracts were combined, washed with brine (3 × 50 ml), dried over magnesium sulfate and filtered. The filtrate was evaporated *in vacuo* leaving the intermediate acid as a yellow oil (0.85 g).

A solution of the yellow oil in toluene (150 ml) was refluxed through a soxhlet containing molecular sieves (3AÅ) for 5 hours. The solution was evaporated *in vacuo* leaving the title compound as a yellow solid. The title compound was recrystallized twice from *n*-butyl chloride to give white needles (0.34 g, 44%)

melting at 114—115°C: pmr ($CDCl_3$) δ 2.03 (2H, m), 2.73 (2H, m), 4.46 (H, m), 5.41 (H, m), 6.19 (H, dd), 7.01 (H, d), 7.14—7.50 (3H, m).

Analysis Calc. for $C_{13}H_{12}Cl_2O_3$

| | | |
|---|---|---|
| Calc.: | C, 54.37; | H, 4.21 |
| Found: | C, 54.31; | H, 4.26 |

$[\alpha]_D^{25} = -6.6°$ (c 0.555; chloroform)

## Example 6

Preparation of (E)-*trans*-6-{2-[3,5-dichloro-4′-fluoro-2-(1,1′-biphenyl)yl]ethenyl}-3,4,5,6-tetrahydro-4-hydroxy-2H-pyran-2-one.

*Step A.* Preparation of 2,4-dichloro-6-methoxybenzaldehyde

By substituting an equimolar amount of methyl iodide for benzyl bromide in Step A of Example 1 there was obtained a corresponding amount of the title compound as a white powder, mp 110°—111°C.

*Step B.* Preparation of N-(2-Hydroxy-1,1-dimethylethyl)-2,4-dichloro-6-methoxybenzamide

A suspension of 2,4-dichloro-6-methoxybenzaldehyde (3 g, 15 mmol) and N-bromosuccinimide (3.6 g, 20 mmol) in carbon tetrachloride (30 ml) was illuminated with a 150 W flood lamp under nitrogen with vigorous stirring on a steam bath for seven minutes. The cloudy mixture was cooled to 0°C, diluted with methylene chloride (30 ml) and treated dropwise with a solution of 2-amino-2-methylpropanol (3 ml, 30 mmol) in methylene chloride (30 ml). The ice bath was removed and the mixture was stirred at 20°C for twenty hours.

The reaction mixture was filtered and the collected solids were washed with additional methylene chloride (50 ml). The clear filtrates were combined and washed with $H_2O$ (100 ml), 5% HCl (100 ml), 5% NaOH (100 ml), $H_2O$ (100 ml) and brine, then dried ($MgSO_4$), filtered and evaporated *in vacuo* to provide the title compound as a white powder (3.6 g, 82%), mp 130—132°C. Crystallization from hexane-toluene (10:8, v:v) provided an analytical sample of title compound, mp 131—132°C.

Analysis Calc. for $C_{12}H_{15}Cl_2NO_3$

| | | | |
|---|---|---|---|
| Calc.: | C, 49.33; | H, 5.18; | N, 4.79 |
| Found: | C, 49.51; | H, 5.27; | N, 4.62. |

*Step C.* Preparation of 2-(2,4-Dichloro-6-methoxyphenyl)-4,4-dimethyl-2-oxazoline

N-(2-Hydroxy-1,1-dimethylethyl)-2,4-dichloro-6-methoxybenzamide (5.5 g, 18.8 mmol) was treated dropwise with thionyl chloride (5.5 ml) and stirred magnetically at 20°C for 30 min. Dry ether (100 ml) was added, the mixture was stirred for an additional one hour and the oxazoline hydrochloride precipitate was collected by filtration. The salt was neutralized with 20% sodium hydroxide to afford an alkaline mixture which was extracted with ether. The ethereal extract was dried ($MgSO_4$) and concentrated to give an oil (3.6 g, 70%), which crystallized on standing, mp 47—50°C.

Analysis for $C_{12}H_{13}Cl_2NO_2$

| | | | |
|---|---|---|---|
| Calc.: | C, 52.57; | H, 4.78; | N, 5.11 |
| Found: | C, 52.60; | H, 4.98; | N, 4.99. |

*Step D.* Preparation of 2-(3,5-Dichloro-4′-fluoro-2-[1,1′-biphenyl]yl)-4,4-dimethyl-2-oxazoline

2-Methoxyphenylmagnesium bromide, prepared from 2-bromoanisole (22.4 g, 120 mmol) and magnesium (2.9 g, 120 mmol) in dry THF (75 ml) was added dropwise to a stirred solution of 2-(2,4-dichloro-6-methoxyphenyl)-4,4-dimethyl-2-oxazoline (100 mmol) in dry THF (150 ml) under $N_2$ at 20°C. Stirring of the solution was continued for 20 hours and then the reaction mixture was quenched by the addition of saturated ammonium chloride solution. The resulting mixture was extracted with ether (2 × 500 ml), dried over $MgSO_4$, filtered and evaporated. The residue was chromatographed on silica gel (ethyl acetate-hexane) to provide title compound (85%), mp 93—95°C.

Analysis for $C_{17}H_{14}Cl_2FNO$

| | | | |
|---|---|---|---|
| Calc.: | C, 60.37; | H, 4.17; | N, 4.14 |
| Found: | C, 60.72; | H, 4.17; | N, 3.89. |

*Step E.* Preparation of 2-(3,5-Dichloro-4′-fluoro-2-[1,1′-biphenyl]yl)-3,4,4-trimethyl-2-oxazolium iodide

A solution of 2-(3,5-dichloro-4′-fluoro-2-[1,1′-biphenyl]yl)-4,4-dimethyl-2-oxazoline (4.6 g, 13.6 mmol)

and methyl iodide (7 ml) in nitromethane (30 ml) was stirred on a steam bath for sixteen hours. The cooled reaction mixture was diluted with dry ether (200 ml) and, after cooling in an ice-bath, the crystalline product was collected to give 6 g (92%) of the title compound, mp 214—216°C dec. Crystallization from acetonitrile-ether (1:3, v:v) provided an analytical sample of the title compound, mp 218—219.5°C dec.

Analysis for $C_{18}H_{17}Cl_2FINO$

| | | | |
|---|---|---|---|
| Calc.: | C, 45.03; | H, 3.57; | N, 2.92 |
| Found: | C, 44.94; | H, 3.47 | N, 2.83. |

*Step F.* Preparation of 3,5-Dichloro-4′-fluoro-1,1′-biphenyl-2-carboxyaldehyde

A vigorously stirred suspension of 2-(3,5-dichloro-4′-fluoro-2-[1,1′-biphenyl]yl)-3,4,4-trimethyl-2-oxazolium iodide (5.9 g, 12.3 mmol) in ethanol (50 ml) was treated portionwise with sodium borohydride (550 mg, 18 mmol). After stirring for two hours at ambient temperature the clear solution was diluted with 3N hydrochloric acid (100 ml) and stirred on a steam bath for two hours. The reaction mixture was then cooled, diluted with $H_2O$ (200 ml) and extracted with ether (300 ml). The ether extract was washed with $H_2O$ (2 × 200 ml) and brine, then dried ($MgSO_4$), filtered and evaporated *in vacuo* to provide 2.72 g (82%) of the title compound, mp 66—68°C. Crystallization from petroleum ether provided an analytical sample of the title compound, mp 73—74°C.

Analysis for $C_{13}H_7Cl_2FO$

| | | |
|---|---|---|
| Calc.: | C, 58.02; | H, 2.62 |
| Found: | C, 58.15; | H, 2.52. |

*Step G.* Preparation of (E)-*trans*-6-[2-(3,5-dichloro-4′-fluoro-2-[1,1′-biphenyl]yl)ethenyl]-3,4,5,6-tetrahydro-4-hydroxy-2H-pyran-2-one

By substituting an equimolar amount of 3,5-dichloro-4′-fluoro-1,1′-biphenyl-2-carboxyaldehyde in place of 2,4-dichloro-6-phenylmethoxybenzaldehyde in the alternate to alternate Step B, of Example 1 and then following the procedures of Step B through F, there was obtained a corresponding amount of the title compound, mp 121—122°C.

Analysis for $C_{19}H_{15}Cl_2FO_3$

| | | |
|---|---|---|
| Calc.: | C, 59.86; | H, 3.97 |
| Found: | C, 59.70; | H, 3.97. |

The *cis* isomer of the title compound was obtained in comparable yield after crystallization from *n*-butyl chloride, mp 107—108°C.

Example 7

Starting with 2-(2,4-Dichloro-6-methoxyphenyl)-4,4-dimethyl-2-oxazoline but substituting equimolar amounts of the following Grignard reagents in place of 4-fluorophenylmagnesium bromide in Step D of Example 6 and following the procedures of Steps D through G there was obtained a corresponding amount of the appropriate end product listed below.

| Grignard Reagent | End Product | mp °C | Calc. | Found |
|---|---|---|---|---|
| 4-chlorophenyl | (E)-*trans*-6-[2-(3,4′,5-trichloro-2-[1,1′-biphenyl]-yl)ethenyl]-3,4,5,6-tetrahydro-4-hydroxy-2H-pyran-2-one | 116.5—118° | C 57.38<br>H 3.80 | C 57.07<br>H 3.85 |

Example 8

Alternate Preparation of 3,5-Dichloro-4′-fluoro-1,1′-biphenyl-2-carboxaldehyde

*Step A.* Preparation of Bis[μ-(Acetato-O:O′)bis-(3,5-dichloro-2[(phenylimino)methyl]phenyl-C,N]dipalladium

A mixture of N-[(2,4-Dichlorophenyl)methylene]benzeneamine (2.5 g, 10 mmole) and palladium (II) acetate (2.24 g, 10 mmole) in acetic acid (50 ml) was heated at reflux for one hour with stirring. The turbid solution was filtered and the filtrate was diluted with water (300 ml) to give the title compound as a red solid (3.9 g, 94%). Crystallization from acetic acid-water (7:1, v:v) provided an analytical sample of the title

compound, mp 203—205°C: pmr ($CDCl_3$) δ 1.73 (3H, S), 6.50 (H, d, J=1.5Hz) 6,97 (2H, m), 7.12 (H, d, J=1.5Hz) 7.33 (3H, m), 8.03 (H, S).

Analysis Calc. for $C_{30}H_{22}Cl_2N_4O_4Pd_2$

| | | | |
|---|---|---|---|
| Calc.: | C, 43.42; | H, 2.67; | N, 3.38 |
| Found: | C, 43.54; | H, 2.59; | N, 3.13. |

*Step B.* Preparation of 3,5-Dichloro-4'-fluoro-1,1'-biphenyl-2-carboxyldehyde

A solution of bis-[μ-(Acetato-0:0')bis-[3,5-dichloro-2-[(phenylimino)methyl]phenyl-C,N]dipalladium (8.29 g, 10 mmole) and triphenylphosphine (21.0 g, 80 mmole) in dry benzene (150 ml) was stirred for 30 minutes at ambient temperature under $N_2$. The 4-fluorophenylmagnesium bromide, prepared from 4-bromofluorobenzene (15.4 g, 88 mmole) and magnesium (1.94 g, 80 mmole) in dry ether (100 ml) under $N_2$ at ambient temperature, was added to the above solution in one portion. The resulting mixture was stirred for one hour at ambient temperature. After the addition of 6N HCl (50 ml) with stirring for one hour, the mixture was filtered. The filtrate was diluted with ether (300 ml) and washed with brine (2 × 100 ml). The organic layer was refiltered to remove more yellow solid and the filtrate, washed with brine (2 × 100 ml), dried over $MgSO_{34}$, filtered and evaporated. The residue was chromatographed on a silica column (1000 g). Elution with ether-hexane (1:39, v:v, 5500 ml) provided a forerun which was discarded. Continued elution with ether-hexane (1:9, v:v, 5700 ml) gave the title compound as a yellow solid (4.5 g, 84%), mp 73—74°C: pmr ($CDCl_3$) δ7.03—7.40 (5H, m), 7.53 (H, d, J=1.5Hz), 10.13 (H, S).

Example 9

Starting with bis[μ-(Acetato-0:0')bis-[3,5-dichloro-2[(phenylimino)methyl]phenyl-C,N]dipalladium but substituting equal amounts of the following Grignard reagents for 4-fluorophenylmagnesium bromide in Step B of Example 8 and following the procedure of Step B there was obtained a corresponding amount of the appropriate end product listed below.

| Grignard | End Product | pmr (δ) |
|---|---|---|
| 4-methylphenyl | 3,5-dichloro-4'-methyl-1,1'-biphenyl-2-carboxaldehyde | 2.40 (3H, S), 7.13–7.40 (5H, m), 7.50 (H, d, J=1.5Hz), 10.06 (H, S) |
| 3-fluorophenyl | 3,5-dichloro-3'-fluoro-1,1'-biphenyl-2-carboxaldehyde | 6.93–7.60 (6H, m), 10.16 (H, S) |

Example 10

By substituting an equimolar amount of the following aldehydes for 2,4-dichloro-6-phenylmethoxy-benzaldehyde in alternate to alternate Step B, Example 1, and then following Steps C through F of Example 1, the corresponding pyran-2H-ones listed below were obtained.

| Starting Aldehyde | End Product | mp | | Analysis Calcd. | Analysis Found |
|---|---|---|---|---|---|
| 3,5-dichloro-4′-methyl-1,1′-biphenyl-2-carboxaldehyde | (E)-*trans*-6-{2-(3,5-dichloro-4′-methyl-2-[2,2′-biphenyl)-[ethenyl}-3,4,5,6-tetrahydro-4-hydroxy-2H-pyran-2-one | 119°–119.5° | C:<br>H: | 63.67<br>4.81 | 63.69<br>4.88 |
| 3,5-dichloro-3′ fluoro-1,1′-biphenyl-2-carboxaldehyde | (E)-*trans*-6-{2-(3,5-dichloro-3′-fluoro-2-[1,1′-biphenyl]yl)-ethenyl}-3,4,5,6-tetrahydro-4-hydroxy-2H-pyran-2-one | 130°–132° | C:<br>H: | 59.86<br>3.97 | 59.75<br>3.94 |

Example 11

Resolution of the Optical Isomers of (±) Trans-6-{2-[2,4-dichloro-6-(4-fluorophenylmethoxy)phenyl]ethyl}-3,4,5,6-tetrahydro-4-hydroxy-2H-pyran-2-one

*Step A.* Preparation and Separation of the Diastereomeric Amides (Diastereomers A and B)

A solution of (±)-*trans*-6-{2-[2,4-dichloro-6-(4-fluorphenylmethoxy)phenyl]ethyl}-3,4,5,6-tetrahydro-4-hydroxy-2H-pyran-2-one (28.3 g, 68 mmole) and l-(−)-α-methylbenzylamine (16.5 g, 136 mmole) in tetrahydrofuran (350 ml) was refluxed for 20 hours. The tetrahydrofuran was removed *in vacuo* and the residue was stirred in ether (500 ml) and the precipitate collected to give diastereomer A which was twice stirred for 15 min. in refluxing ether (500 ml) to yield a colorless solid (13.0 g, 36%) which melted at 185°—188°C.

*Step B.* Preparation of (+) Trans-6-{2-[2,4-dichloro-6-(4-fluorophenylmethoxy)phenyl]ethyl}-3,4,5,6-tetrahydro-4-hydroxy-2H-pyran-2-one

By substituting an equimolar amount of diastereomer A from Step A above for diastereomer A in Step B of Example 5A and following the procedure described therein, there was obtained a corresponding amount of the title compound which was recrystallized from *n*-butylchloride-pet ether (4:3, v:v), mp 133°—135°C; pmr ($CDCl_3$) δ 1.53—2.20 (5H, m); 2.66 (H, m), 2.93 (2H, m), 4.36 (H, m), 4.73 (H, m), 5.04 (2H, s), 6.85 (H, d), 7.03—7.53 (5H, m).

Analysis Calc. for $C_{20}H_{19}Cl_2FO_4$

| | | |
|---|---|---|
| Calc.: | C, 58.12; | H, 4.63 |
| Found: | C, 58.25; | H, 4.71. |

$[\alpha]_D^{25}$ +17.8° (C, 1.0; chloroform)

Example 12

Preparation of (+)-(E)-(3R*, 5S*)-7-(3,5-dichloro-4′-fluoro[1,1′-biphenyl]-2yl)-3,5-dihydroxy-6-heptenoic acid, ammonium salt.

*Step A.* Preparation and Separation of Diastereomeric Amides

By substituting an equimolar amount of (±) - *trans* - (E) - 6 - {2 - [3,5 - dichloro - 4′ - fluoro(1,1′ - biphenyl] - 2 - yl)ethenyl} - 3,4,5,6 - tetrahydro - 4 - hydroxy - 2H - pyran - 2 - one for (±) - *trans* - 6 - {2 - [2,4 - dichloro - 6 - (4 - fluorophenylmethoxy)phenyl]ethyl} - 3,4,5,6 - tetrahydro - 4 - hydroxy - 2H - pyran - 2 - one in Step A of Example 11 and following the procedure described therein there was obtained a corresponding amount of Diastereomer A as colorless crystals which melted at 128.5°—129°C.

*Step B.* Preparation of (+)-(E)-(3R*, 5S*)-7-{3,5-dichloro-4′-fluoro[1,1′-biphenyl]-2-yl}-3,5-dihydroxy-6-heptenoic acid, ammonium salt

Diastereomer A (6.2 g, 12.3 mmole) of Step A was dissolved in 95% ethanol (600 ml) containing 1N NaOH (60 ml, 60 mmole) and the solution was refluxed for 16 hours. The solvent was removed *in vacuo* and the residue was suspended in ice water (200 ml) and ether (500 ml) and subsequently acidified with 3N HCl (50 ml). The ether layer was washed successively with ice-cold 1N HCl (200 ml), brine (2 × 200 ml), dried over $MgSO_4$, and filtered. Anhydrous ammonia was bubbled through the cold etherial solution for 2 min. Vigorous stirring was then continued at 20°C for 1 hour and then the mixture was cooled slowly to *ca* 5° C. Filtration provided the title compound as tiny colorless needles (4.3 g, 84%), mp 105°—108°C dec. pmr ($d_6$-DMSO) δ 1.15 (H, m), 1.41 (H, m), 1.99 (H, dd), 2.14 (H, dd), 3.66 (H, m), 4.11 (H, dd), 5.52 (H, dd) 6.38 (H, d), 7.23—7.42 (5H, m), 7.69 (H, d).

Analysis for $C_{19}H_{20}Cl_2FNO_4$

| | | | |
|---|---|---|---|
| Calc.: | C, 54.82; | H, 4.84; | N, 3.36 |
| Found: | C, 55.13; | H, 4.98; | N, 3.07. |

$[\alpha]_D^{27}$ = + 10.75° (C, 1.6; water).

**Claims for the Contracting States: BE CH DE FR GB IT LI LU NL SE**

1. Compounds having the structure

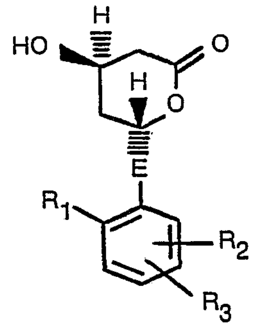

wherein
E is —CH=CH— or —$CH_2$—$CH_2$—
$R_1$ is phenyl substituted by halo and/or $C_{1-4}$ alkyl, or $OR_4$ in which $R_4$ is phenyl $C_{1-3}$ alkyl unsubstituted or substituted with halo,
$R_2$ and $R_3$ are independently hydrogen, halo, or $C_{1-4}$ alkyl or the corresponding dihydroxy acids resulting from the hydrolytic opening of the lactone ring, and the pharmaceutically acceptable salts of said acids, or the lower alkyl and phenyl, dimethylamino or acetylamino-substituted lower alkyl esters of said dihydroxy acids; all said compounds being the 4-*R* enantiomer of the *trans* racemate whose structure is shown above.

2. The compound of claim 1 in which $R_1$ is halophenyl-$C_{1-3}$-alkoxy or halophenyl, $R_2$ is halo in the 2-position, $R_3$ is halo in the 4-position.

3. The compounds of claim 2 wherein $R_2$ and $R_3$ are chloro.

4. The compounds of claim 3 wherein E is —CH=CH— and $R_1$ is 4-fluorophenyl.

5. A process for preparing the compounds of claim 1, which comprises the aldol condensation of the dianion of acetoacetic ester with an aldehyde of the structure

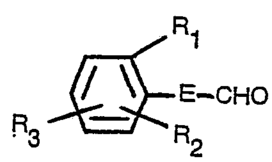

followed by reduction of the 3-keto group to a hydroxyl, saponification of the ester, lactonization of the carboxyl with the 6-hydroxyl, if desired, reducing any double bonds in the bridging group E, and separating the *cis* and *trans* racemates and separation thereform of the 4 (R) trans enantiomers.

6. A hypocholesterolemic, hypolipemic, pharmaceutical composition comprising a pharmaceutical carrier and an effective hypocholesterolemic, hypolipemic amount of a compound of claim 1.

**Claims for the Contracting State: AT**

1. A process for preparing compounds having the structure

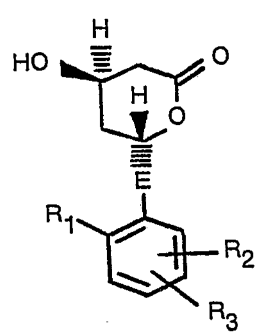

wherein
E is —CH=CH— or —$CH_2$—$CH_2$—
$R_1$ is phenyl substituted by halo and/or $C_{1-4}$ alkyl, or $OR_4$ in which $R_4$ is phenyl $C_{1-3}$ alkyl unsubstituted or substituted with halo,
$R_2$ and $R_3$ are independently hydrogen, halo, or $C_{1-4}$ alkyl or the corresponding dihydroxy acids resulting from the hydrolytic opening of the lactone ring, and the pharmaceutically acceptable salts of said acids, or the lower alkyl and phenyl, dimethylamino or acetylamino-substituted lower alkyl esters of said

dihydroxy acids; all said compounds being the 4-*R* enantiomer of the *trans* racemate whose structure is shown above
which comprises the aldol condensation of the dianion of acetoacetic ester with an aldehyde of the structure

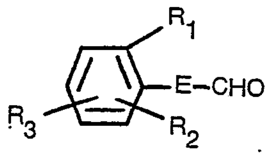

followed by reduction of the 3-keto group to a hydroxyl, saponification of the ester, lactonization of the carboxyl with the 6-hydroxyl, if desired, reducing any double bonds in the bridging group E, and separating the *cis* and *trans* racemates and separation thereform of the 4 (R) trans enantiomers.

2. The process of claim 1 for the preparation of the compounds wherein $R_1$ is halophenyl-$C_{1-3}$-alkoxy or halophenyl, $R_2$ is halo in the 2-position, $R_3$ is halo in the 4-position.

3. The process of claim 2 for the preparation of the compounds wherein $R_2$ and $R_3$ are halo.

4. The process of claim 3 for the preparation of the compounds wherein E is —CH=CH— and $R_1$ is fluorophenyl.

**Patentansprüche für die Vertragsstaaten: BE CH DE FR GB IT LI LU NL SE**

1. Verbindungen mit der Struktur

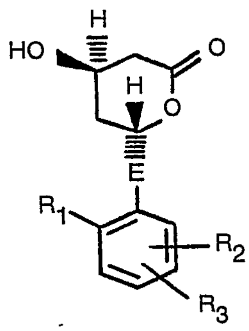

worin
E —CH=CH— oder —$CH_2$—$CH_2$— ist,
$R_1$ durch Halogen und/oder $C_{1-4}$-Alkyl substituiertes Phenyl, oder $OR_4$ ist, wobei $R_4$ Phenyl-$C_{1-3}$-alkyl darstellt, das unsubstituiert oder durch Halogen substituiert ist, und
$R_2$ und $R_3$ unabhängig voneinander Wasserstoff, Halogen oder $C_{1-4}$-Alkyl sind,
oder die entsprechenden Dihydroxysäuren, die bei der hydrolytischen Öffnung des Lactonrings entstehen, und die pharmazeutisch annehmbaren Salze der genannten Säuren, oder die Niedrigalkyl- und Phenyl-, dimethylamino- oder acetylamino-substituierten Niedrigalkylester der genannten Dihydroxysäuren; wobei all die genannten Verbindungen das 4-R-Enantiomer des trans-Racemats, dessen Struktur oben dargestellt ist, sind.

2. Die Verbindungen des Anspruchs 1, in welchen, $R_1$ Halogenphenyl-$C_{1-3}$-alkoxy oder Halogenphenyl ist, $R_2$ Halogen in der 2-Stellung ist und $R_3$ Halogen in der 4-Stellung ist.

3. Die Verbindungen des Anspruchs 2, worin $R_2$ und $R_3$ Chlor sind.

4. Die Verbindungen des Anspruchs 3, worin E —CH=CH— ist und $R_1$ 4-Fluorphenyl ist.

5. Ein Verfahren zur Herstellung der Verbindungen des Anspruchs 1, welches die Aldolkondensation des Dianions von Acetessigester mit einem Aldehyd der Struktur

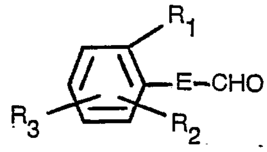

und die anschließende Reduktion der 3-Ketogruppe zu einem Hydroxyl, die Versiefung des Esters, die Lactonisierung des Carboxyls mit dem 6-Hydroxyl, gewünschtenfalls die Reduktion einer etwaigen Doppelbindung in der brückenbildenden Gruppe E, und die Trennung der cis- und trans-Racemate sowie die Trennung der 4(R)-trans-Enantiomeren davon umfaßt.

6. Eine hypocholesterinämische, hypolipämische, pharmazeutische Zusammensetzung, enthaltend einen pharmazeutischen Träger und eine hypocholesterinämisch und hypolipämisch wirksame Menge einer Verbindung des Anspruchs 1.

**Patentansprüche für den Vertragsstaat: AT**

1. Ein Verfahren zur Herstellung von Verbindungen mit der Struktur

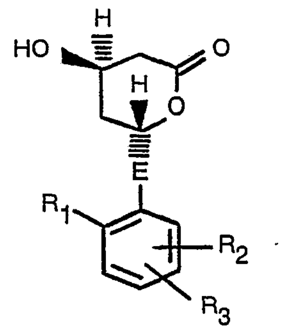

worin

E —CH=CH— oder —$CH_2$—$CH_2$— ist,

$R_1$ durch Halogen und/oder $C_{1-4}$-Alkyl substituiertes Phenyl, oder $OR_4$ ist, wobei $R_4$ Phenyl-$C_{1-3}$-alkyl darstellt, das unsubstituiert oder durch Halogen substituiert ist, und

$R_2$ und $R_3$ unabhängig voneinander Wasserstoff, Halogen oder $C_{1-4}$-Alkyl sind,

oder der entsprechenden Dihydroxysäuren, die bei der hydrolytischen Öffnung des Lactonrings entstehen, und der pharmazeutisch annehmbaren Salze der genannten Säuren, oder der Niedrigalkyl- und Phenyl-, dimethylamino- oder acetylamino-substituierten Niedrigalkylester der genannten Dihydroxysäuren; wobei all die genannten Verbindungen das 4-R-Enantiomer des trans-Racemats, dessen Struktur oben dargestellt ist, sind; welches die Aldolkondensation des Dianions von Acetessigester mit einem Aldehyd der Struktur

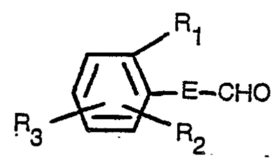

und die anschließende Reduktion der 3-Ketogruppe zu einem Hydroxyl, die Versiefung des Esters, die Lactonisierung des Carboxyls mit dem 6-Hydroxyl, gewünschtenfalls die Reduktion einer etwaigen Doppelbindung in der brückenbildenden Gruppe E, und die Trennung der cis- und trans-Racemate sowie die Trennung der 4(R)-trans-Enantiomeren davon umfaßt.

2. Das Verfahren des Anspruchs 1 für die Herstellung der Verbindungen, in welchen $R_1$ Halogenphenyl-$C_{1-3}$-alkoxy oder Halogenphenyl ist, $R_2$ Halogen in der 2-Stellung ist und $R_3$ Halogen in der 4-Stellung ist.

3. Das Verfahren des Anspruchs 2 für die Herstellung der Verbindungen, worin $R_2$ und $R_3$ Halogen sind.

4. Das Verfahren des Anspruchs 3 für die Herstellung der Verbindungen, worin E —CH=CH— ist und $R_1$ Fluorphenyl ist.

**Revendications pour les Etats contractants: BE CH DE FR GB IT LI LU NL SE**

1. Composés de structure

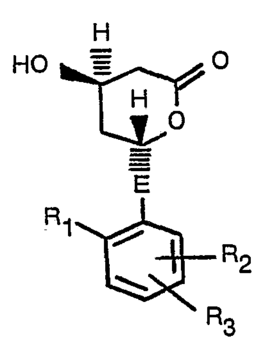

dans laquelle

E est —CH=CH— ou —$CH_2$—$CH_2$—

$R_1$ est un radical phényle à substitution halo et/ou alkyle en $C_{1-4}$, ou bien $OR_4$ dans lequel $R_4$ est un phénylalkyle en $C_{1-3}$ non substitué ou à substituant halo,

$R_2$ et $R_3$ représentent chacun indépendamment un atome d'hydrogène, un radical halo ou alkyle en $C_{1-4}$ et les acides dihydroxyliques correspondants provenant de l'ouverture hydrolytique du noyau de lactone, et les sels pharmaceutiquement acceptables desdits acides, ou bien les esters alkyliques inférieurs substitués par alkyle, phényle, diméthylamino ou acétylamino desdits acides dihydroxyliques; tous ces composés étant les 4-*R*-énantiomères du *trans*-racémate dont la structure est indiquée ci-dessus.

2. Composés selon la revendication 1, dans lesquels $R_1$ est un radical halophényl-alcoxy en $c_{1-3}$ ou halopényle, $R_2$ est un radical halo en position 2, $R_3$ est un radical halo en position 4.

3. Composés selon la revendication 2, dans lesquels $R_2$ et $R_3$ sont des radicaux chloro.

4. Composés selon la revendication 3, dans lesquels E est —CH=CH— et $R_1$ est le 4-fluorophényle.

5. Procédé de préparation des composés selon la revendication 1, qui consiste à effecteur une condensation Aldol du dianion de l'ester acétoacétique avec un aldéhyde de formule

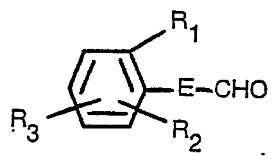

puis à réduire le groupe 3-céto en un hydroxyle, à saponifier l'ester, à lactoniser le carboxyle avec le 6-hydroxyle, éventuellement à réduire des liaisons doubles dans le groupe de pontage E, à séparer les racémates cis et trans et à séparer de ceux-ci des transénantiomères 4 (R).

6. Composition pharmaceutique hypocholestérolémique et hypolémique qui comprend unvéhicule pharmaceutique et une quantité efficace hypocholestérolémique et hypolipémique d'un composé selon la revendication 1.

**Revendications pour l'Etat contractant: AT**

1. Procédé de préparation de composés de structure

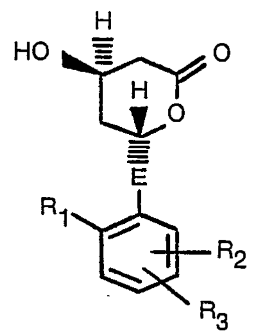

dans laquelle

E est —CH=CH— ou —$CH_2$—$CH_2$—

$R_1$ est un radical phényle à substitution halo et/ou alkyle en $C_{1-4}$, ou bien $OR_4$ dans lequel $R_4$ est un phénylalkyle en $C_{1-3}$ non substitué ou à substituant halo,

$R_2$ et $R_3$ représentent chacun indépendamment un atome d'hydrogène, un radical halo ou alkyle en $C_{1-4}$ et les acides dihydroxyliques correspondants provenant de l'ouverture hydrolytique du noyau de lactone, et les sels pharmaceutiquement acceptables desdits acides, ou bien les esters alkyliques inférieurs substitués par alkyle, phényle, diméthylamino ou acétylamino desdits acides dihydroxyliques; tous ces composés étant les 4-*R*-énantiomères du *trans*-racémate dont la structure est indiquée ci-dessus, qui consiste à effecteur une condensation Aldol du dianion de l'ester acétoacétique avec un aldéhyde de formule

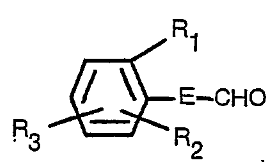

puis à réduire le groupe 3-céto en un hydroxyle, à saponifier l'ester, à lactoniser le carboxyle avec le 6-hydroxyle, à réduire les liaisons doubles éventuelles dans le groupe de pontage E, à séparer les racémates cis et trans et à séparer de ceux-ci les transénantiomères 4 (R).

2. Procédé selon la revendication 1, pour la préparation des composés dans lesquels: $R_1$ est halophényl-alcoxy en $C_{1-3}$ ou halophényle, $R_2$ est halo en position 2, $R_3$ est halo en position 4.

3. Procédé selon la revendication 2, pour la préparation des composés dans lequel $R_2$ et $R_3$ sont des radicaux halo.

4. Procédé selon la revendication 3, pour la préparation de composés dans lesquels E est —CH=CH— et $R_1$ est le fluorophényle.